# FASCICULE DE BREVET EUROPEEN

(11) **EP 4 486 418 B1**
(45) Date de publication et mention de la délivrance du brevet: **11.02.2026**
(21) Numéro de dépôt: 23707736.7
(22) Date de dépôt: 01.03.2023
(51) Int. Cl.: A61M 11/04, A61M 15/00, A61M 15/06

(54) **DISPOSITIF MÉDICAL D'ADMINISTRATION SÉCURISÉE DE MÉDICAMENT SOUS FORME FLUIDE**
MEDIZINISCHE VORRICHTUNG ZUR SICHEREN VERABREICHUNG EINES ARZNEIMITTELS IN FLÜSSIGER FORM
MEDICAL DEVICE FOR SECURELY ADMINISTERING A DRUG IN FLUID FORM

(30) Priorité: 03.03.2022 FR 2201854
(43) Date de publication de la demande: 08.01.2025
(73) Titulaire: ASSISTANCE PUBLIQUE HOPITAUX DE PARIS, 75012 Paris 12 (FR)
(72) Inventeur: BERLIN, Ivan, 75012 Paris (FR); BIHAN, Kevin, 92290 Chatenay-Malabry (FR)
(74) Mandataire: Cabinet Camus Lebkiri
(86) Numéro de dépôt international: PCT/EP2023/055111
(87) Numéro de publication internationale: WO 2023/166025

(56) Documents cités:
- WO-A1-2016/028544
- WO-A1-2017/083541
- WO-A1-2018/032667
- WO-A1-2019/016740
- WO-A1-2019/136437
- WO-A1-2020/194297

## Description

### DOMAINE TECHNIQUE DE L'INVENTION

La présente invention concerne un dispositif médical d'administration sécurisée, par inhalation, d'un médicament sous forme fluide.

L'invention trouve des applications dans le domaine médical, en particulier, pour l'administration de médicaments par voie respiratoire haute et basse avec un effet recherché systémique ou local.

### ARRIERE-PLAN TECHNOLOGIQUE DE L'INVENTION

On entend par médicament toute substance ou composition présentée comme possédant des propriétés curatives ou préventives à l'égard des maladies humaines ou animales, ainsi que toute substance ou composition pouvant être utilisée chez l'homme ou chez l'animal ou pouvant leur être administrée, en vue d'établir un diagnostic médical ou de restaurer, corriger ou modifier leurs fonctions physiologiques en exerçant une action pharmacologique, immunologique ou métabolique (www.legifrance.gouv.fr/codes/id/LEGISCTA000006171363). La partie du médicament responsable de son action pharmacologique est le principe actif.

La réponse thérapeutique à un médicament dépend des facteurs déterminant son absorption, sa distribution, son effet sur les sites d'action, son métabolisme et son élimination. La réponse thérapeutique d'un patient à l'administration d'un médicament comporte des effets souhaités, correspondant à l'objectif thérapeutique, et des effets non-souhaités, appelés effets indésirables, plus ou moins prévisibles.

Un médicament, peut être administré à un patient par différentes voies, appelées voies d'administration. La voie d'administration d'un médicament conditionne son lieu d'absorption et la vitesse à laquelle le médicament entre dans la circulation sanguine. Le lieu d'absorption est un facteur déterminant de la biodisponibilité du médicament. La biodisponibilité est la quantité du médicament disponible dans l'organisme pour arriver sur les sites d'actions et aboutissant à un effet pharmacologique. La biodisponibilité dépend de la quantité de principe actif absorbée sur la surface d'absorption, de la vitesse d'absorption et de l'entrée dans la circulation sanguine. Par définition, un médicament administré par voie intraveineuse a une biodisponibilité égale à 100%. Un médicament administré par d'autres voies a généralement une biodisponibilité inférieure en raison de l'absorption partielle à travers des tissus (cutanés, muqueuses) et de son métabolisme tissulaire (notamment hépatique) parfois intense. La biodisponibilité d'un médicament dépend notamment du type de surface d'absorption, de la taille de cette surface d'absorption et des caractéristiques physicochimiques dudit médicament. Plus la surface d'absorption est grande, plus la quantité de médicament absorbée est élevée et, par conséquent, plus la biodisponibilité est élevée. De plus, l'évitement, après absorption du médicament, de son métabolisme précoce (principalement gastro-entérique et hépatique) augmente sa biodisponibilité.

La voie d'administration peut être une voie invasive comme, par exemple, l'injection intra-veineuse, intra-artérielle, sous-cutanée, intra-musculaire, intra-articulaire, etc. Au contraire, la voie d'administration peut être une voie non-invasive comme, par exemple, l'absorption à travers les muqueuses (muqueuses nasales, oculaires, sublinguales, orales, gastrointestinales, rectales) ou l'absorption à travers la peau (voie transdermique).

Une des voies d'administration les plus performantes est la voie intra-veineuse. Cependant, non seulement cette voie est invasive, mais en plus elle nécessite que le patient soit en milieu médicalisé avec la présence d'un personnel formé et compétent.

Une des voies d'administration les plus courantes est la voie d'administration orale par ingestion du médicament. Si cette voie présente l'avantage que le patient peut s'autoadministrer le médicament, elle présente aussi l'inconvénient d'entraîner un délai d'apparition de l'effet thérapeutique relativement long (de l'ordre de plusieurs dizaines de minutes à plusieurs heures) voire réduit en raison du ralentissement de la vitesse et la quantité de médicament atteignant la circulation veineuse en raison du délai physiologique relatif au transit gastro-intestinal et d'un métabolisme tissulaire (notamment hépatique) parfois important .

La voie d'administration sublinguale avec absorption par la muqueuse buccale est aussi une voie d'administration courante. Cependant, la surface d'absorption est petite, ce qui limite la quantité du principe actif absorbée.

Une autre des voies d'administration couramment utilisée est la voie inhalée. Cette voie présente l'avantage d'être non-invasive et est généralement utilisée pour des actions locales, essentiellement bronchiques, via un aérosol. Un aérosol est un ensemble de particules solides (aérosol-doseur) ou liquides (nébuliseur) dont le diamètre est suffisamment petit pour qu'elles restent en suspension dans un gaz ou un mélange gazeux (air). Cette voie inhalée nécessite l'utilisation de dispositifs médicaux adaptés. Les dispositifs médicaux utilisés pour l'administration par voie inhalée, actuellement sur le marché, sont essentiellement les nébuliseurs, ou nébulisateurs. Un nébuliseur comporte un compresseur qui pousse de l'air ou de l'oxygène dans une chambre où le médicament - sous forme liquide - est transformé en une brume pouvant être inhalée au moyen d'un masque ou d'un embout buccal ou nasal. Cette brume, ou aérosol, a l'avantage de faire arriver le principe actif rapidement sur les sites d'action (bronches) avec un relativement faible passage du principe actif dans la circulation générale veineuse. Cependant, les particules de l'aérosol délivrées par un nébuliseur sont en moyenne comprises entre 3 et 5 µm de diamètre. Le principe actif véhiculé par les particules de l'aérosol atteint le site de liaison, site d'action bronchique. Toutefois, une grande partie du principe actif (environ 80-85 %) reste dans la cavité buccale et l'œsophage, réduisant ainsi la quantité de principe actif atteignant les sites d'action bronchiques. Cette voie d'administration vise les voies respiratoires hautes (qui s'étendant de la cavité nasale jusqu'aux bronches de large diamètre). L'effet systémique - voire même local - d'un médicament délivré par un nébuliseur est donc réduit par la captation du principe actif du médicament dans les tissus de la paroi bronchique. On estime que seules moins de 20% des particules les plus fines (<2 µm de diamètre) atteignent le poumon profond. C'est pourquoi cette modalité d'administration est inadaptée pour une action systémique des médicaments.

Le nébuliseur présente, en outre, l'inconvénient d'être bruyant et encombrant, et donc inapproprié pour un usage en ambulatoire. Il est également couteux et contraignant en termes d'utilisation et de sécurisation car il nécessite une surveillance régulière de son bon fonctionnement et la préparation du médicament à administrer par un personnel formé.

On connait par exemple les dispositifs médicaux décrits dans les demandes WO 2017/083541 A1, WO 2016/028544 A1 et WO 2019/136437 A qui permettent l'administration par voie respiratoire de médicaments aérosolisés.

Les autres dispositifs médicaux utilisés actuellement pour l'administration par voie inhalée sont les aérosols-doseurs (ou sprays) et les inhalateurs de poudres, notamment pour les patients asthmatiques. Bien que ces derniers soient adaptés à une utilisation en ambulatoire, ces aérosols présentent l'inconvénient - à l'instar des nébuliseurs - de générer des gouttelettes de dimensions relativement élevées qui restent, pour leur majorité, dans le dispositif médical lui-même ou la sphère buccale et l'œsophage.

Il existe un besoin d'un dispositif d'administration par voie inhalée permettant une auto-administration des médicaments pour une action locale broncho-alvéolaire ou, au contraire, avec une biodisponibilité systémique importante et utilisable dans des indications diverses.

### RESUME DE L'INVENTION

Pour répondre aux problèmes évoqués ci-dessus des dispositifs médicaux pour l'administration de médicaments par voie inhalée, le demandeur propose un dispositif médical sécurisé comprenant un réservoir hermétique équipé d'un dispositif d'aérosolisation et une unité de commande contrôlant la dose de médicament contenue dans le réservoir et pouvant être inhalée par le patient.

Dans la suite de la description, le terme « médicament » sera employé pour désigner un médicament sous une forme fluide, contenu dans le réservoir du dispositif médical. Ce médicament sous forme fluide, appelé aussi solution médicamenteuse, comporte un principe actif et des excipients en solution.

En fonction des caractéristiques spécifiques du principe actif et de sa forme galénique (c'est-à-dire la forme pharmaceutique dans laquelle le principe actif est présenté), la voie d'administration par inhalation a deux avantages : a) l'inhalation permet l'arrivée du principe actif au niveau des bronchioles et alvéoles pulmonaires et un passage immédiat et direct dans la circulation artérielle (système des veines pulmonaires) ; b) l'obtention d'un effet local pulmonaire si le principe actif et sa forme galénique ne traversent qu'a minima les membranes des petites bronches ou alvéolaires. La première approche permet une action immédiate et systémique, la deuxième un effet local pulmonaire ciblant les infections ou néoplasies avec localisation exclusive ou majoritaire pulmonaire. En effet, l'accès des médicaments jusqu'au niveau bronchiolaire/alvéolaire à des doses thérapeutiques permettra une action locale ou systémique supérieure à ce qui existe actuellement.

On appelle « aérosolisation » la technique de dispersion, dans l'air ou dans un espace clos, d'un liquide ou d'une solution sous la forme de fines particules.

Selon un premier aspect, l'invention concerne un dispositif médical d'administration sécurisée par voie respiratoire de médicament comprenant :
- un réservoir hermétique renfermant le médicament sous une forme fluide,
- un boîtier sur lequel est monté le réservoir,
- un embout buccal en liaison fluidique avec le réservoir et par lequel le médicament peut être introduit dans la cavité buccale d'un patient,
- une unité de commande traitant des données et paramètres relatifs à l'administration du médicament et contrôlant la posologie et recueillant des informations de sécurité d'emploi dudit médicament inhalé par le patient, caractérisé en ce qu'il comprend en outre un dispositif d'aérosolisation du médicament par élévation de sa température, générant un aérosol micro-particulé dudit médicament, inhalable par le patient, l'aérosol micro-particulé comportant des particules d'un diamètre inférieur à 2 µm dispersées dans l'air, et en ce que ledit dispositif d'aérosolisation comprend un élément de chauffage au moins partiellement logé à l'intérieur du réservoir et rétractable hors du réservoir.

Ce dispositif permet de sécuriser l'administration du médicament tant en termes de contrôle des administrations et des posologies qu'en termes de manipulation, car le patient n'a accès qu'au réservoir hermétiquement clos : il n'a pas accès au médicament situé à l'intérieur du réservoir dans un milieu stérile et l'ajustement des posologies est contrôlé et paramétré par le dispositif lui-même de façon à limiter ou faciliter la délivrance des doses de médicament nécessaires. De plus, ce dispositif étant portatif et ergonomique (c'est-à-dire de dimensions suffisamment réduites pour permettre une utilisation manuelle facile), il permet une auto-administration par le patient en ambulatoire.

Selon l'invention, le dispositif d'aérosolisation comprend un élément de chauffage rétractable au moins partiellement logé à l'intérieur du réservoir, le médicament étant aérosolisé par élévation de la température de l'élément de chauffage.

Chauffer la solution médicamenteuse réduit le diamètre des particules, ce qui permet au médicament aérosolisé d'atteindre les bronchioles et alvéoles pulmonaires en quantité suffisante pour une biodisponibilité et une efficacité optimales et cela par opposition aux dispositifs médicaux actuellement disponibles. Ainsi la réduction de la taille des particules par aérosolisation par élévation de la température permet une diffusion importante dans les voies respiratoires basses jusqu'au niveau alvéolaire. Ainsi, des médicaments à visée systémique peuvent arriver, grâce à un passage direct dans la circulation artérielle pulmonaire, directement dans le ventricule cardiaque gauche et être distribués par la circulation artérielle, aboutissant à une diffusion systémique extra-rapide (< 1-2 minutes) sans perte du principe actif. En effet, la voie d'administration respiratoire permet de s'affranchir d'une dégradation précoce du principe actif en évitant un métabolisme gastro-entérique, hépatique (i.e. effet de premier passage hépatique) et même le métabolisme tissulaire périphérique.

Le médicament selon sa définition légale, utilisé dans le dispositif médical de l'invention peut engendrer un effet systémique ou un effet local pulmonaire.

Outre les caractéristiques qui viennent d'être évoquées dans le paragraphe précédent, le dispositif médical selon un aspect de l'invention peut présenter une ou plusieurs caractéristiques complémentaires parmi les suivantes, considérées individuellement ou selon toutes les combinaisons techniquement possibles :
- l'élément de chauffage comprend une résistance chauffante comportant une partie supérieure logée dans le réservoir et une partie inférieure logée dans le boîtier, ladite partie inférieure étant entraînée en déplacement par au moins un moteur.
- l'élément de chauffage est au moins partiellement creux de sorte à laisser passer un flux d'air en son sein.
- le dispositif d'aérosolisation comprend un générateur d'ultrasons, le médicament étant aérosolisé par sonication.
- les données et paramètres relatifs à l'administration du médicament comprennent des informations de sécurité d'emploi du médicament.
- le réservoir comporte un bouchon supérieur et un bouchon inférieur, aptes à fermer hermétiquement le réservoir, l'ouverture et la fermeture du bouchon inférieur étant commandées par une rotation du dispositif d'aérosolisation, l'ouverture et la fermeture du bouchon supérieur étant commandées par une rotation du bouchon inférieur.
- le bouchon inférieur comporte une surface inférieure comportant une encoche prévue pour recevoir une extrémité du dispositif d'aérosolisation, une surface supérieure comportant un redan prévu pour commander le bouchon supérieur, et une paroi latérale comportant au moins un ergot de verrouillage.
- le boîtier comporte une glissière pour guider l'insertion du réservoir sur ledit boitier et/ou son retrait.
- il comprend un lecteur d'étiquettes, connecté à l'unité de commande, pour lire une étiquette d'identification apposée sur le réservoir.
- l'étiquette d'identification comporte un QR code et le lecteur d'étiquettes est un lecteur de QR codes.
- il comprend un dispositif de détection du niveau ou de la quantité du médicament dans le réservoir, connecté à l'unité de commande.
- il comprend une batterie électrique rechargeable logée dans le boîtier et alimentant en énergie électrique au moins l'unité de commande.
- il comporte un écran d'affichage des données relatives à l'administration du médicament et/ou aux données de paramétrage, connecté à l'unité de commande.

### BREVE DESCRIPTION DES FIGURES

D'autres avantages et caractéristiques de l'invention apparaîtront à la lecture de la description qui suit, illustrée par les figures dans lesquelles :
La figure 1 représente schématiquement les voies respiratoires d'un être humain ;
La figure 2 représente une vue schématique en perspective du dispositif médical selon l'invention ;
La figure 3 représente des vues schématiques en coupe, de côté et de profil, du dispositif médical selon l'invention ;
La figure 4 représente des vues de face, de côté, de dessus et de dessous du dispositif médical selon l'invention ;
La figure 5 représente diverses vues de face et en perspective de l'élément de chauffage selon certains modes de réalisation de l'invention ;
La figure 6 représente diverses vues de face, de dessus, de dessous et en perspective du réservoir selon certains modes de réalisation de l'invention ;
La figure 7 représente diverses vues du bouchon inférieur du réservoir selon certains modes de réalisation de l'invention ; et
La figure 8 représente diverses vues de face et en perspective du réservoir du dispositif médical lors de l'insertion de l'élément de chauffage.

### DESCRIPTION DETAILLEE

Un exemple de réalisation d'un dispositif médical d'administration d'un médicament, dans lequel l'accès au médicament et la dose inhalable par le patient sont sécurisés, est décrit en détail ci-après, en référence aux dessins annexés. Cet exemple illustre les caractéristiques et les avantages de l'invention. Il est toutefois rappelé que l'invention ne se limite pas à cet exemple.

Sur les figures, les éléments identiques sont repérés par des références identiques. Pour des questions de lisibilité des figures, les échelles de taille entre éléments représentés ne sont pas respectées.

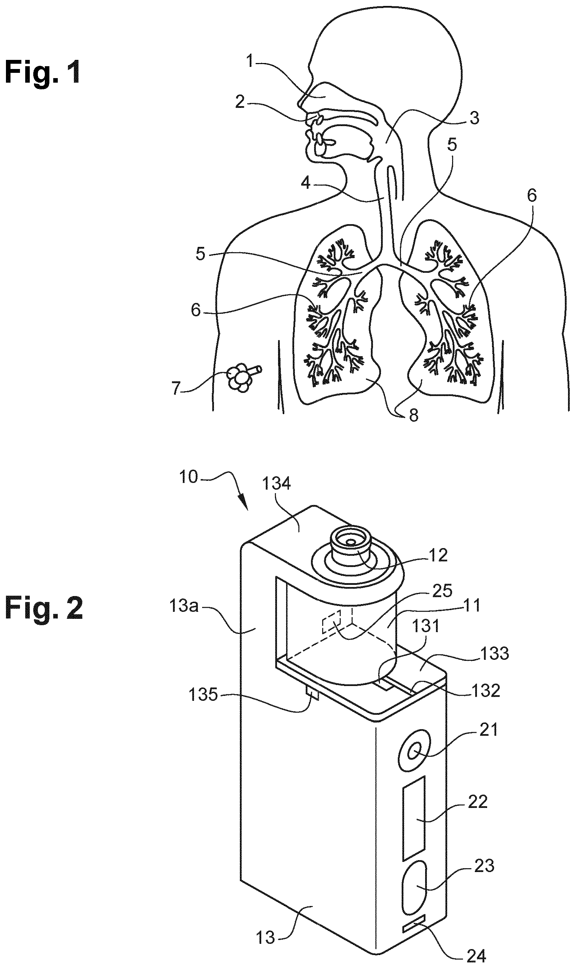

Un exemple du dispositif médical sécurisé selon l'invention est représenté sur la figure 2. Ce dispositif médical 10 comporte un boîtier 13 sur lequel est monté un réservoir 11 amovible. Le réservoir 11 est un récipient fermé hermétiquement, contenant la solution médicamenteuse à administrer au patient. Ce réservoir 11 est rempli de médicament par un professionnel (du domaine médical ou pharmaceutique) et scellé par ce professionnel de sorte que le patient ne puisse avoir accès audit médicament. Bien qu'il n'ait pas accès au contenu du réservoir 11, le patient a accès au réservoir lui-même et peut monter le réservoir sur le boîtier 13 et/ou le démonter. Le montage du réservoir 11 est réalisé par coulissement d'une tête de coulissement (placée sous ledit réservoir et non visible sur les figures) dans une glissière 132 formée sur une surface intermédiaire 133 du boîtier 13. Un élément de blocage 131, tel qu'un téton de sécurité ou un poussoir à ressort, par exemple positionné au voisinage de la glissière 132, assure le blocage du réservoir 11 lorsque celui-ci est dans sa position d'utilisation. En position d'utilisation, le réservoir est positionné entre la surface intermédiaire 133 et la surface supérieure 134 du boîtier 13. Le démontage du réservoir est réalisé de façon inverse en débloquant l'élément de blocage 131 et en faisant coulisser le réservoir 11 jusqu'à l'entrée de la glissière 132, à l'extrémité de la surface intermédiaire 133.

Le dispositif médical 10 comporte, de plus, un embout buccal 12 fixé sur une surface supérieure 134 du boîtier 13. Cet embout buccal 12 est d'une forme classique, par exemple similaire à celle des embouts des cigarettes électroniques ou celle des embouts des inhalateurs buccaux. Dans une variante, non représentée sur les figures, l'embout buccal 12 peut être monté directement sur le réservoir 11, la surface supérieure 134 du boîtier 13 ne recouvrant alors pas totalement le réservoir. Quelle que soit la variante (fixé sur le boîtier ou fixé sur le réservoir), l'embout buccal 12 est en liaison fluidique avec le réservoir 11 lorsque celui-ci est dans sa position d'utilisation, c'est-à-dire qu'il assure la circulation du médicament micro-aérosolisé du réservoir jusqu'à la cavité buccale du patient.

Le dispositif médical 10 comporte également un dispositif d'aérosolisation du médicament, logé au moins partiellement dans le réservoir 11. Ce dispositif d'aérosolisation est conçu pour transformer le médicament en solution fluide en un médicament aérosolisé pouvant être inhalé par le patient. Le dispositif d'aérosolisation est adapté pour générer un aérosol microparticulé, c'est-à-dire un aérosol sous forme de microparticules. On appelle « microparticules » ou « microgouttelettes » des particules ou gouttelettes de très petites dimensions par rapport aux particules ou gouttelettes produites dans un aérosol classique. Dans le domaine médical, une microparticule est une particule dont le diamètre est inférieur à 2 µm.

Dans un mode de réalisation, le dispositif d'aérosolisation comporte un générateur d'ultrasons permettant d'aérosoliser le médicament par sonication. La sonication est adaptée, par exemple, pour les principes actifs qui sont susceptibles de subir une modification structurelle au-delà d'une température ambiante définie, par exemple, comme une température inférieure à 30 ou 40°C. La sonification du médicament permet de transformer le médicament en microparticules aérosolisées sans augmentation de la température. Dans ce mode de réalisation, le générateur d'ultrasons est logé à l'intérieur du réservoir 11 et commandé par l'unité de commande décrite ultérieurement.

Dans un autre mode de réalisation, décrit en détail par la suite, le dispositif d'aérosolisation comprend un élément de chauffage, par exemple une résistance chauffante 15, permettant de chauffer le médicament de sorte à transformer le médicament sous la forme de microparticules aérosolisées. En effet, une solution aérosolisée, soit par élévation de sa température, soit par sonification, est formée de microparticules ou microgouttelettes, c'est-à-dire des gouttelettes de très petites dimensions par rapport aux gouttelettes ou particules des aérosols classiques. Et, plus les gouttelettes sont petites, plus elles se déplacent facilement et profondément dans les voies respiratoires basses d'un patient.

L'aérosolisation par sonification peut être une alternative à l'aérosolisation par chauffage, par exemple si la chauffe est contre-indiquée ou inadaptée pour un médicament. Quel que soit son mode de réalisation, le dispositif d'aérosolisation du dispositif selon l'invention permet d'atteindre les organes tissus les plus profonds des voies respiratoires, comme les alvéoles pulmonaires

Pour bien comprendre les effets, d'un point de vue respiratoire, du dispositif d'aérosolisation équipant le dispositif médical de l'invention, un exemple de voies respiratoires d'un être humain est représenté sur la figure 1. Les voies respiratoires s'étendent des cavités nasales 1 et buccale 2 jusqu'aux alvéoles pulmonaires 7 en traversant le pharynx 3, la trachée 4, les bronches 5 et les bronchioles 6. L'air inhalé par un être humain entre donc dans le système respiratoire par les cavités nasales 1 et/ou la cavité buccale 2 puis traverse le pharynx 3, la trachée 4, les bronches 5 et les bronchioles 6 pour finir dans les alvéoles 7 des poumons 8 où l'échange gazeux se fait à travers les membranes alvéolaires et endothéliales. Les dispositifs médicaux d'administration par voie inhalée, connus à ce jour, permettent au mieux au principe actif d'atteindre les surfaces bronchiques (5). Le dispositif médical selon l'invention permet une administration respiratoire plus profonde : il permet au médicament d'atteindre les surfaces bronchiolaires et alvéolaires.

Dans certaines situations thérapeutiques, comme dans le traitement de la douleur ou dans les épisodes aigus de pathologies chroniques (liste non exhaustive), l'obtention d'un effet thérapeutique rapide, par exemple inférieur à 10 minutes, voire inférieur à 1 minute, est primordiale. Le dispositif selon l'invention donne non seulement l'accès au médicament directement à la circulation artérielle, mais en plus, la surface d'absorption alvéolaire étant particulièrement étendue (de l'ordre de 50m² selon des estimations physiologiques ), cela assure l'absorption d'une grande quantité de médicament dans la circulation artérielle, assurant une biodisponibilité proche de 100 % voire supérieure à 100 % (la référence étant la concentration plasmatique du médicament dans le sang veineux). Le dispositif selon l'invention assure donc une biodisponibilité et une vitesse d'absorption relativement élevées et un accès rapide du principe actif aux tissus cibles, comme par exemple le système nerveux central. L'effet thérapeutique peut être obtenu en quelques secondes ou dizaines de secondes). En termes de tolérance, certains effets indésirables (notamment ceux dits 'dose-dépendants') sont susceptibles d'être réduits pour les médicaments dont la réduction de la dose administrée sera permise par l'amélioration de leur biodisponibilité par rapport aux voies existantes. En effet, en améliorant la diffusion alvéolaire du médicament et du fait de l'importante surface alvéolaire, la dose inhalée nécessaire à l'obtention de l'effet thérapeutique est réduite par rapport aux formes pharmaceutiques actuelles.

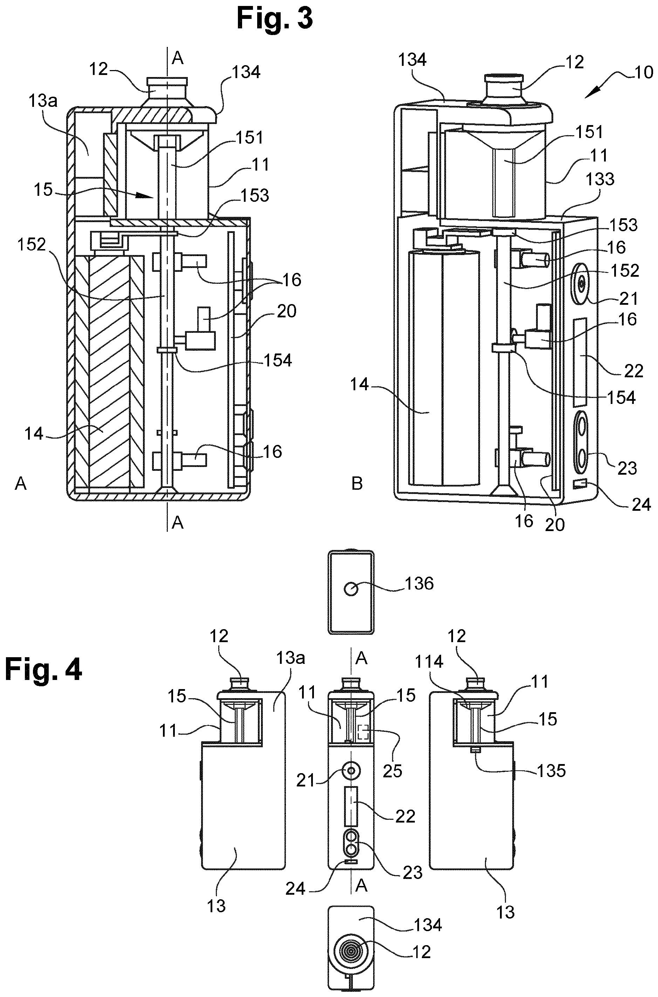

Comme cité précédemment, le mode de réalisation préféré de l'aérosolisation du médicament est l'intégration d'une résistance chauffante 15 dans le réservoir 11 du dispositif médical de l'invention. Des exemples d'une résistance chauffante 15, montée dans le boîtier 13 du dispositif médical de l'invention, sont représentés sur les figures 3 et 4. Cette résistance chauffante 15, appelée plus simplement résistance, comporte une partie supérieure 151 et une partie inférieure 152. La partie supérieure 151 vient se loger dans le réservoir 11 et la partie inférieure 152, entraînée par au moins un moteur, assure des mouvements mécaniques (insertion/rétractation et rotation), comme expliqué par la suite. La partie supérieure 151 de la résistance comporte un élément chauffant logé dans un fourreau formant une gaine de protection dudit élément chauffant. L'élément chauffant peut être formé, par exemple, d'une couche de coton ou de silice entourant un fil de résistance. Cette partie supérieure 151 assure par contact la montée en température de la solution médicamenteuse contenue dans le réservoir 11. Elle peut assurer une température allant jusqu'à 200°C. La partie inférieure 152 de la résistance 15 comporte un support sur lequel est fixée la partie supérieure 151. Cette partie inférieure 152 est conçue pour être mobile longitudinalement le long d'un axe longitudinal AA et par rotation à l'intérieur du boîtier 13.

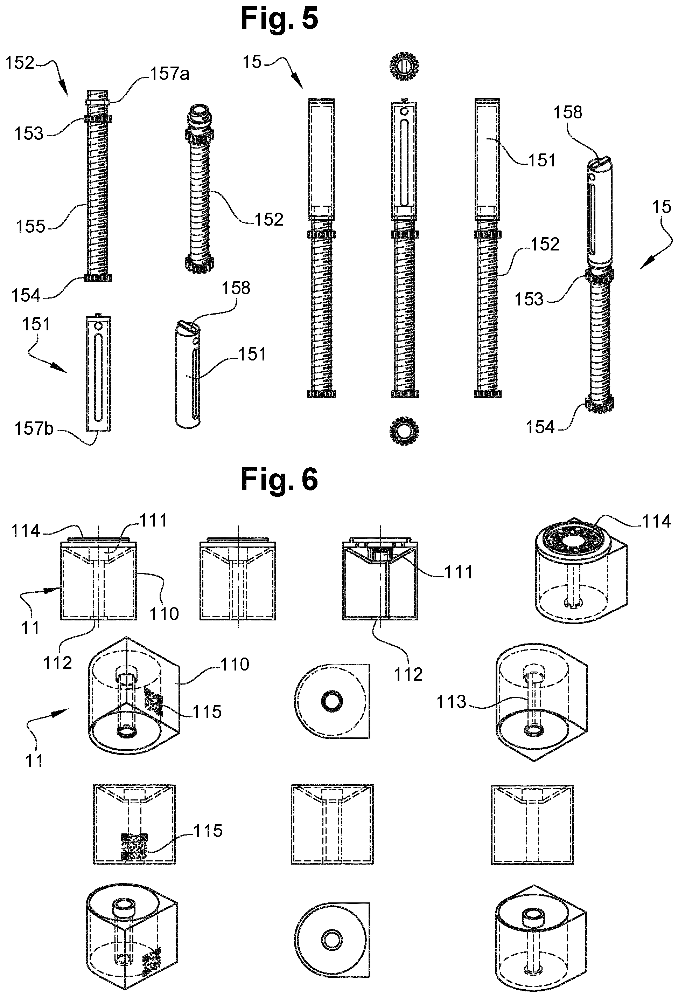

Un exemple de la résistance 15 est représenté plus en détail sur la figure 5. Cette figure 5 montre la partie supérieure 151 montée sur la partie inférieure 152. La partie inférieure 152 présente une forme tubulaire ayant une surface externe pourvue d'une dentelure 155. Cette dentelure 155, par exemple une crémaillère ou un pas de vis, est adaptée pour être engrenée dans un ou plusieurs pignons 153, 154. Un ou plusieurs moteurs 16 sont logés dans le boîtier 13 pour entraîner le/les pignons 153, 154 en rotation qui, à leur tour, entraînent la partie inférieure 152 de la résistance en translation. Par exemple, au moins un premier moteur peut assurer une rotation de la résistance 15 autour de l'axe AA (dans un sens horaire et dans un sens anti-horaire) et au moins un second moteur peut assurer la translation de la résistance 15 suivant l'axe AA pour permettre l'insertion et le retrait de la partie supérieure 151 dans le réservoir 11.

Comme la partie supérieure 151 de la résistance 15 est montée solidaire de la partie inférieure 152, la partie supérieure 151 est entraînée en translation par la partie inférieure 152. L'ensemble formé des moteurs, des pignons et de la partie inférieure 152 permettent ainsi de translater la partie supérieure 151 du boîtier 13 vers le réservoir 11, ou inversement du réservoir 11 vers le boîtier 13. La résistance 15 est ainsi rétractable. Cette rétractabilité de la résistance 15 permet l'incorporation de ladite résistance au sein du réservoir 11 de façon sécurisée car la résistance 15 entre en contact avec le médicament sans aucune manipulation humaine. Tout utilisateur non professionnel, et notamment le patient, est donc en mesure de changer facilement le réservoir 11 sans jamais être en contact avec le médicament lui-même.

Dans certains modes de réalisation, la partie supérieure 151 de la résistance est montée de façon fixe sur la partie inférieure 152. Dans d'autres modes de réalisation, la partie supérieure 151 de la résistance est montée de façon amovible sur la partie inférieure 152, ce qui permet de retirer la partie supérieure chauffante 151, par exemple pour la nettoyer ou pour l'échanger lorsqu'elle est défectueuse ou usée. Dans ces modes de réalisation où la partie supérieure est amovible, le fourreau de la partie supérieure 151 peut être muni d'un pas de vis interne 157b, engrenant avec un pas de vis externe 157a positionné à l'extrémité de la partie inférieure 152. D'autres moyens de maintien amovible de la partie supérieure 151 sur la partie inférieure 152 peuvent bien entendu être envisagés comme une goupille, un circlip, une agrafe, etc.

La figure 6 représente un exemple d'un réservoir 11 destiné à recevoir le médicament. Ce réservoir 11 comporte un récipient 110 hermétique, conçu pour que le médicament y soit chauffé ou « soniqué ». Le récipient 110 est donc adapté pour recevoir, de préférence de façon centrale, la partie supérieure 151 de la résistance 15. Pour cela, un espace longitudinal est prévu au sein du récipient 110 pour recevoir la partie supérieure 151 de la résistance 15. Dans l'exemple de la figure 6, un système de guidage 113 est installé au centre du récipient 110 pour guider la partie supérieure 151 de la résistance lors de son introduction et/ou son retrait du réservoir. Ce système de guidage 113 peut être, par exemple, une goulotte à l'intérieur de laquelle glisse la partie supérieure 151 lorsque celle-ci est introduite dans le récipient 110, ou extraite dudit récipient ; ce système de guidage 113 peut alternativement être un rail de guidage le long duquel se translate la partie supérieure 151.

Le réservoir 11 comporte un couvercle perforé 114 sur lequel est monté l'embout buccal 12. Il comporte également un bouchon inférieur 112 et un bouchon supérieur 111 assurant la fermeture hermétique du réservoir 11 lorsque celui-ci est en position de manutention. La position de manutention est, par définition, la position contraire de la position d'utilisation : en position de manutention, le réservoir (plein ou vide) est démonté du boîtier 13 et peut être manipulé ; en position d'utilisation, le réservoir est fixé sur le boîtier 13 et peut accueillir la partie supérieure de la résistance 15. Ainsi, lorsque le réservoir 11 est en position de manutention, les bouchons supérieur 111 et inférieur 112 sont dans une position fermée de sorte que le réservoir est hermétiquement clos. Au contraire, lorsque le réservoir 11 est en position d'utilisation, le bouchon inférieur 112 et le bouchon supérieur 111 sont en position ouverte de sorte à permettre l'aérosolisation. Pour des raisons de sécurité, l'ouverture de ces bouchons supérieur et inférieur est commandée par la résistance 15, elle-même commandée par une unité de commande 20.

L'ouverture et la fermeture du bouchon inférieur 112 sont commandées par une rotation de la partie supérieure de la résistance 15, tandis que l'ouverture et la fermeture du bouchon supérieur 111 sont commandées par une rotation du bouchon inférieur 112 après que ces derniers aient été mis en contact l'un avec l'autre, après élévation de la résistance 15 au sein du réservoir 11.

Le bouchon supérieur 111 est monté sous le couvercle perforé 114, en regard du système de guidage 113. Le bouchon supérieur 111 comporte, sur une face inférieure, une encoche (non visible sur les figures) destinée à recevoir un redan 116 (partie en saillie) du bouchon inférieur 112.

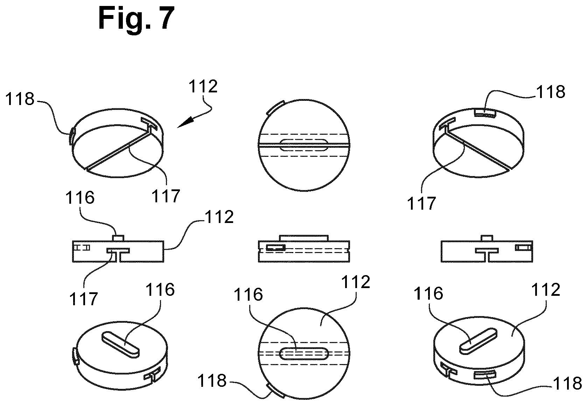

Le bouchon inférieur 112, dont un exemple est représenté sur les dessins de la figure 7, est monté en regard du système de guidage 113 et du bouchon supérieur 111. Le bouchon inférieur 112 comporte, sur une face inférieure, une encoche 117 et, sur une face supérieure, un redan 116. L'encoche 117 du bouchon inférieur 112 est une rainure destinée à recevoir un redan 158 positionné à l'extrémité de la partie supérieure 151 de la résistance 15. Le redan 116 du bouchon inférieur 112 est une saillie allongée s'étendant suivant le diamètre du bouchon et destinée à s'insérer dans une encoche (non visible sur les figures) du bouchon supérieur 111.

Le bouchon inférieur 112 comporte des ergots de verrouillage 118 positionnés latéralement sur le pourtour dudit bouchon inférieur et assurant le verrouillage/déverrouillage dudit bouchon sur le récipient 110. De façon similaire, le bouchon supérieur 111 comporte des ergots de verrouillage (non visibles sur les figures) positionnés latéralement sur le pourtour dudit bouchon supérieur et assurant le verrouillage/déverrouillage dudit bouchon sur le récipient 110.

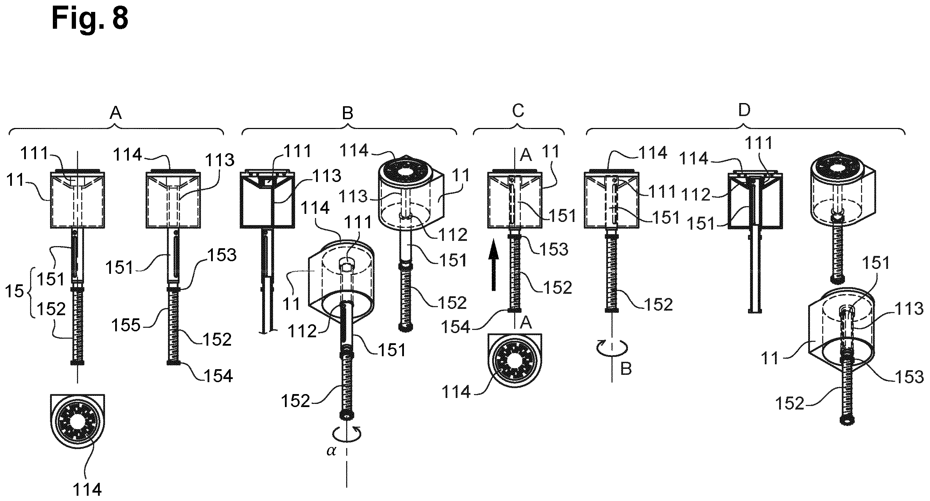

Comme énoncé ci-dessus, l'ouverture des bouchons supérieur 111 et inférieur 112 est commandée par la résistance 15 de sorte à sécuriser l'ouverture et/ou la fermeture du réservoir 11, notamment lorsque celui-ci est rempli du médicament. Un exemple d'insertion de la résistance 15 dans le réservoir 11 est représenté, en plusieurs étapes, sur la figure 8. Ces dessins montrent la résistance 15 dans ses différentes positions depuis sa position « rétractée » (à gauche de la figure) jusqu'à sa position « de chauffage » (à droite de la figure). Ces dessins de la figure 8 montrent notamment la résistance 15 :
- en position rétractée (dessin A) : la résistance 15 est totalement logée dans le boîtier 13 ;
- en position de déverrouillage du bouchon inférieur 112 (dessin B) : la résistance 15 est tournée d'un angle α (inférieur à 180°) de sorte que son redan 158 logé dans l'encoche 117 du bouchon inférieur 112 débloque les ergots de verrouillage 116 dudit bouchon inférieur ;
- en position d'élévation (dessin C) : la résistance 15 est translatée le long de l'axe AA vers le réservoir 11 de sorte que sa partie supérieure 151 soit totalement insérée dans le réservoir 11 et que le redan 116 du bouchon inférieur 112 se loge dans l'encoche du bouchon supérieur 111 ;
- en position de déverrouillage du bouchon supérieur 111 (dessin D) : la résistance 15 est tournée d'un angle β, de sens opposé à l'angle α, de sorte à entraîner la rotation du redan 116 du bouchon inférieur 112 logé dans l'encoche du bouchon supérieur, ce qui débloque les ergots de verrouillage dudit bouchon supérieur.

Une fois que le bouchon supérieur 111 est déverrouillé, le récipient 110 n'est plus hermétiquement clos. La résistance 15 peut être mise en chauffe pour que le médicament soit transformé en aérosol microparticulé. Le bouchon supérieur 111 étant déverrouillé, les perforations du couvercle 114 sont libres, c'est-à-dire ouvertes ; l'aérosol peut donc s'évacuer vers le patient, via ces perforations et l'embout buccal 12. Le patient peut alors utiliser le dispositif médical selon ses besoins.

Pour assurer la circulation du flux d'air nécessaire à la formation de l'aérosol microparticulé, la résistance 15 est au moins partiellement creuse. Le flux d'air peut ainsi circuler depuis une entrée d'air modulable/ajustable 136 jusqu'à l'embout buccal 12, en traversant le boîtier 13 via la partie inférieure creuse 152 de ladite résistance et le réservoir 11 via la partie creuse supérieure 151 de la résistance 15.

Le dispositif médical 10 peut comporter différents composants, représentés sur les figures 2, 3 et 4, destinés à améliorer le dispositif et/ou faciliter son utilisation. Il peut comporter notamment une batterie électrique 14, rechargeable ou non, de préférence logée dans le boîtier 13, et alimentant en énergie électrique au moins l'unité de commande et les moteurs. Le dispositif médical peut comporter aussi une prise de connexion 24 pour recevoir un chargeur d'alimentation (notamment lorsque la batterie est rechargeable) et/ou un dispositif de stockage de données (par exemple pour télécharger des données).

Le dispositif médical 10 peut comporter également un bouton d'allumage 21, un écran d'affichage de données 22 et un ou plusieurs bouton(s) de navigation 23 pour permettre l'accès à la visualisation (sur l'écran 22) des différentes données. L'écran d'affichage 22 peut afficher des données relatives, par exemple, à la date et l'heure de prise du médicament, au nom du médicament, à sa date de péremption, à la température de chauffe de la résistance, au nombre d'utilisations ou de doses possibles du médicament, au nombre d'utilisations ou de doses restantes, au volume du médicament restant dans le réservoir, à la durée de vie de la résistance, à des indications thérapeutiques ou techniques, etc. Le bouton d'allumage 21, ou bouton ON/OFF, permet au patient de commander l'aérosolisation du médicament en sécurisant le risque d'allumage spontané de la résistance. Ce bouton d'allumage 21 peut permettre soit une commande d'allumage et une commande d'extinction de l'aérosolisation, soit uniquement la commande d'allumage, l'extinction étant réalisée de façon automatique par l'unité de commande 20. Pour éviter un allumage spontané, l'aérosolisation peut être initiée par une série d'appuis consécutifs dans un intervalle défini (par exemple trois répétitions en moins de 3secondes).

L'unité de commande 20, logée dans le boîtier 13, comporte un microprocesseur, une carte mémoire et/ou une carte électronique. L'unité de commande 20 est conçue pour mémoriser diverses données, comme des données relatives au médicament et/ou au patient. Elle est conçue également pour gérer la dose d'aérosol absorbable par le patient, la dose absorbable pouvant être, par exemple, une quantité maximale quotidienne, un nombre de prises, une quantité maximale unitaire (par prise), etc.

L'unité de commande est, de plus, conçue pour commander la température de chauffe de la résistance 15. En effet, l'unité de commande peut contrôler la température de la résistance en fonction de données préalablement définies, par le personnel médical ou le personnel pharmaceutique. La température de chauffe peut, par exemple, être définie en fonction des caractéristiques du médicament et/ou de la pathologie du patient. Cette température de chauffe peut, être indiquée dans l'étiquette 115 apposée sur le réservoir 11 et décrite plus loin.

L'unité de commande 20 peut aussi être conçue pour contrôler le flux d'air sortant du réservoir ainsi que le volume du médicament et/ou le nombre de doses restant dans le réservoir. Pour cela, l'unité de commande peut intégrer un algorithme calculant le volume restant ou le nombre de doses restant, en fonction du volume initial du médicament et du nombre de doses déjà administrées. En variante, l'unité de commande 20 peut être connectée à un dispositif de détection du niveau du médicament restant dans le réservoir. Ce dispositif de détection, ou capteur de niveau, est connecté à l'unité de commande 20. Ce capteur de niveau peut être, par exemple, un capteur photoélectrique, un capteur laser, un capteur photo-optique, un capteur capacitif, un capteur ultrasonique, etc.

D'une façon générale, l'unité de commande 20 intègre et mémorise de nombreux paramètres relatifs au médicament lui-même, à la pathologie du patient, à la technique d'administration du médicament et/ou à la sécurité d'utilisation du dispositif médical 10. L'unité de commande contrôle également la mise en œuvre de ces paramètres ainsi que la mise en place ou la rétractation de la résistance, la mise en chauffe de ladite résistance et l'ouverture/fermeture du bouchon supérieur 111 et inférieur 112.

Le dispositif médical 10 peut comporter également un évent 135, réalisé dans au moins une des parois du boîtier 13, pour assurer la ventilation à l'intérieur dudit boîtier afin d'éviter toute surchauffe de l'unité de commande et/ou des autres composants dudit dispositif médical.

Selon certains modes de réalisation, le dispositif médical 10 comporte un lecteur d'étiquettes 25, par exemple logé dans la partie supérieure 13a du boîtier 13, et connecté à l'unité de commande 20. Ce lecteur d'étiquettes 25 est prévu pour lire des étiquettes apposées sur les réservoirs 11. En effet, chaque réservoir 11 peut être muni d'une étiquette d'identification permettant l'accès à de nombreuses données, générales et/ou spécifiques au patient, relatives au médicament contenu dans le réservoir. Les données auxquelles l'étiquette donne accès peuvent être, par exemple, des précautions d'utilisation du médicament, la date de péremption de ce dernier, la température de chauffe conseillée, la durée d'une inhalation, l'intervalle maximum/minimum entre deux inhalations, la dose de médicament contenu dans une inhalation, etc. L'étiquette peut être, par exemple un code barre ou un QR code. Le lecteur d'étiquettes est alors, respectivement, un lecteur de code-barres ou un lecteur de QR codes. Dans l'exemple représenté sur les figures 2 et 4, le lecteur d'étiquettes 25 est logé dans la partie supérieure 13a du boîtier 13 et l'étiquette 115 est apposée sur la face du réservoir 11 de sorte que l'étiquette 115 soit en regard du lecteur d'étiquettes 25. Cet ensemble étiquette et lecteur d'étiquettes permet non seulement un téléchargement aisé des données mais assure également une sécurisation de ces données, notamment lorsque ces données contiennent les informations relatives au médicament et les paramètres optimaux d'utilisation (exemple : date de péremption, nom du médicament, températures maximales autorisées, doses maximales par prise/quotidienne, déclaration directe d'effets indésirables au centre de pharmacovigilance concerné, renvoi optionnel au résumé des caractéristiques du produit (RCP)).

Bien que décrit à travers un certain nombre d'exemples, variantes et modes de réalisation, le dispositif médical selon l'invention comprend divers variantes, modifications et perfectionnements qui apparaîtront de façon évidente à l'homme du métier, étant entendu que ces variantes, modifications et perfectionnements font partie de la portée de l'invention tel que définie par les revendications.

## Revendications

1. Dispositif médical (10) d'administration sécurisée par voie respiratoire d'un médicament comprenant :
- un réservoir (11) hermétique renfermant le médicament sous une forme fluide,
- un boîtier (13) sur lequel est monté le réservoir (11),
- un embout buccal (12) en liaison fluidique avec le réservoir (11) et par lequel le médicament peut être introduit dans la cavité buccale d'un patient, et
- une unité de commande (20) traitant des données et paramètres relatifs à l'administration du médicament et contrôlant la posologie dudit médicament inhalé par le patient,
- **caractérisé en ce qu'**il comprend en outre un dispositif d'aérosolisation (15) du médicament par élévation de sa température, générant un aérosol micro-particulé dudit médicament, inhalable par le patient, l'aérosol micro-particulé comportant des particules d'un diamètre inférieur à 2 µm dispersées dans l'air,
- et **en ce que** ledit dispositif d'aérosolisation (15) comprend un élément de chauffage au moins partiellement logé à l'intérieur du réservoir (11) et rétractable hors du réservoir.

2. Dispositif selon la revendication 1, **caractérisé en ce que** l'élément de chauffage comprend une résistance chauffante (15) comportant une partie supérieure (151) logée dans le réservoir (11) et une partie inférieure (152) logée dans le boîtier (13), ladite partie inférieure étant entraînée en déplacement par au moins un moteur (16).

3. Dispositif selon la revendication 1 ou 2, **caractérisé en ce que** l'élément de chauffage (15) est au moins partiellement creux de sorte à laisser passer un flux d'air en son sein.

4. Dispositif selon l'une quelconque des revendications 1 à 3, **caractérisé en ce que** les données et paramètres relatifs à l'administration du médicament comprennent des informations de sécurité d'emploi du médicament.

5. Dispositif selon l'une quelconque des revendications 1 à 4, **caractérisé en ce que** le réservoir (11) comporte un bouchon supérieur (111) et un bouchon inférieur (112), aptes à fermer hermétiquement le réservoir (11), l'ouverture et la fermeture du bouchon inférieur (112) étant commandées par une rotation du dispositif d'aérosolisation (15), l'ouverture et la fermeture du bouchon supérieur (111) étant commandées par une rotation du bouchon inférieur (112).

6. Dispositif selon la revendication 5, **caractérisé en ce que** le bouchon inférieur (112) comporte :
- une surface inférieure comportant une encoche (117) prévue pour recevoir une extrémité du dispositif d'aérosolisation (158),
- une surface supérieure comportant un redan (116) prévu pour commander le bouchon supérieur (111), et
- une paroi latérale comportant au moins un ergot de verrouillage (118).

7. Dispositif selon l'une quelconque des revendications 1 à 6, **caractérisé en ce que** le boîtier (13) comporte une glissière (132) pour guider l'insertion du réservoir (11) sur ledit boitier et/ou son retrait.

8. Dispositif selon l'une quelconque des revendications 1 à 7, **caractérisé en ce qu'**il comprend un lecteur d'étiquettes (25), connecté à l'unité de commande (20), pour lire une étiquette d'identification (115) apposée sur le réservoir.

9. Dispositif selon la revendication 8, **caractérisé en ce que** l'étiquette d'identification (115) comporte un QR code et le lecteur d'étiquettes (25) est un lecteur de QR codes.

10. Dispositif selon l'une quelconque des revendications 1 à 9, **caractérisé en ce qu'**il comprend un dispositif de détection du niveau ou de la quantité du médicament dans le réservoir, connecté à l'unité de commande.

11. Dispositif selon l'une quelconque des revendications 1 à 10, **caractérisé en ce qu'**il comprend une batterie électrique rechargeable (14) logée dans le boîtier (13) et alimentant en énergie électrique au moins l'unité de commande.

12. Dispositif selon l'une quelconque des revendications 1 à 11, **caractérisé en ce qu'**il comporte un écran d'affichage (22) des données relatives à l'administration du médicament et/ou aux données de paramétrage, connecté à l'unité de commande (20).

## Patentansprüche

1. Medizinische Vorrichtung (10) zur sicheren Verabreichung eines Medikaments über die Atemwege, umfassend:
- einen hermetisch verschlossenen Behälter (11), der das Medikament in flüssiger Form enthält,
- ein Gehäuse (13), an dem der Behälter (11) angebracht ist,
- ein Mundstück (12), das in Fluidverbindung mit dem Behälter (11) steht und über das das Medikament in die Mundhöhle eines Patienten eingeführt werden kann, und
- eine Steuereinheit (20), die Daten und Parameter bezüglich der Verabreichung des Medikaments verarbeitet und die Dosierung des vom Patienten inhalierten Medikaments kontrolliert,
- **dadurch gekennzeichnet, dass** sie außerdem eine Aerosolisierungsvorrichtung (15) zur Aerosolisierung des Medikaments durch Erhöhung seiner Temperatur umfasst, die ein mikropartikuläres Aerosol des Medikaments erzeugt, das vom Patienten inhaliert werden kann, wobei das mikropartikuläre Aerosol Partikel mit einem Durchmesser von weniger als 2 µm enthält, die in der Luft dispergiert sind,
- und **dadurch gekennzeichnet, dass** die Aerosolisierungsvorrichtung (15) ein Heizelement umfasst, das zumindest teilweise im Inneren des Behälters (11) untergebracht und aus dem Behälter herausziehbar ist.

2. Vorrichtung nach Anspruch 1, **dadurch gekennzeichnet, dass** das Heizelement einen Heizwiderstand (15) umfasst, der einen oberen Teil (151), der im Behälter (11) untergebracht ist, und einen unteren Teil (152) umfasst, der im Gehäuse (13) untergebracht ist, wobei der untere Teil durch mindestens einen Motor (16) angetrieben wird.

3. Vorrichtung nach Anspruch 1 oder 2, **dadurch gekennzeichnet, dass** das Heizelement (15) zumindest teilweise hohl ist, so dass ein Luftstrom durch es hindurchströmen kann.

4. Vorrichtung nach einem der Ansprüche 1 bis 3, **dadurch gekennzeichnet, dass** die Daten und Parameter bezüglich der Verabreichung des Medikaments Informationen zur Anwendungssicherheit des Medikaments umfassen.

5. Vorrichtung nach einem der Ansprüche 1 bis 4, **dadurch gekennzeichnet, dass** der Behälter (11) einen oberen Verschluss (111) und einen unteren Verschluss (112) aufweist, die den Behälter (11) hermetisch verschließen können, wobei das Öffnen und Schließen des unteren Verschlusses (112) durch eine Drehung der Aerosolisierungsvorrichtung (15) gesteuert wird und das Öffnen und Schließen des oberen Verschlusses (111) durch eine Drehung des unteren Verschlusses (112) gesteuert wird.

6. Vorrichtung nach Anspruch 5, **dadurch gekennzeichnet, dass** der untere Verschluss (112) Folgendes umfasst:
- eine Unterseite mit einer Aussparung (117) zur Aufnahme eines Endes der Aerosolisierungsvorrichtung (158),
- eine Oberseite mit einer Verzahnung (116) zur Steuerung des oberen Verschlusses (111) und
- eine Seitenwand mit mindestens einem Verriegelungsstift (118).

7. Vorrichtung nach einem der Ansprüche 1 bis 6, **dadurch gekennzeichnet, dass** das Gehäuse (13) eine Führungsschiene (132) aufweist, um das Einsetzen des Behälters (11) in das Gehäuse und/oder dessen Entnahme zu führen.

8. Vorrichtung nach einem der Ansprüche 1 bis 7, **dadurch gekennzeichnet, dass** sie einen mit der Steuereinheit (20) verbundenen Etikettenleser (25) zum Lesen eines auf dem Behälter angebrachten Identifikationsetiketts (115) umfasst.

9. Vorrichtung nach Anspruch 8, **dadurch gekennzeichnet, dass** das Identifikationsetikett (115) einen QR-Code aufweist und der Etikettenleser (25) ein QR-Code-Lesegerät ist.

10. Vorrichtung nach einem der Ansprüche 1 bis 9, **dadurch gekennzeichnet, dass** sie eine mit der Steuereinheit verbundene Vorrichtung zum Erfassen des Füllstands oder der Menge des Medikaments im Behälter umfasst.

11. Vorrichtung nach einem der Ansprüche 1 bis 10, **dadurch gekennzeichnet, dass** sie eine wiederaufladbare elektrische Batterie (14) umfasst, die im Gehäuse (13) untergebracht ist und mindestens die Steuereinheit mit elektrischer Energie versorgt.

12. Vorrichtung nach einem der Ansprüche 1 bis 11, **dadurch gekennzeichnet, dass** sie einen Bildschirm (22) zur Anzeige von Daten bezüglich der Verabreichung des Medikaments und/oder von Einstellungsdaten umfasst, der mit der Steuereinheit (20) verbunden ist.

## Claims

1. A medical device (10) for the safe administration of a drug via the respiratory tract, comprising:
- a sealed tank (11) containing the drug in fluid form,
- a casing (13) to which the tank (11) is mounted,
- a mouthpiece (12) in fluid connection with the tank (11) and through which the drug can be introduced into a patient's buccal cavity, and
- a control unit (20) processing data and parameters relating to the administration of the drug and controlling dosage of said drug inhaled by the patient,
- **characterised in that** it further comprises an aerosolising device (15) for aerosolising the drug by raising its temperature, generating a micro-particulate aerosol of said drug inhalable by the patient, the micro-particulate aerosol including particles with a diameter of less than 2 µm dispersed in air,
- and **in that** said aerosolising device (15) comprises a heating element at least partially housed inside the tank (11) and retractable from of the tank.

2. The device according to claim 1, **characterised in that** the heating element comprises a heating resistor (15) including an upper part (151) housed in the tank (11) and a lower part (152) housed in the casing (13), said lower part being movably driven by at least one motor (16).

3. The device according to claim 1 or 2, **characterised in that** the heating element (15) is at least partially hollow so as to allow an air flux to pass therewithin.

4. The device according to any of claims 1 to 3, **characterised in that** the data and parameters relating to the administration of the drug comprise safety information for use of the drug.

5. The device according to any of claims 1 to 4, **characterised in that** the tank (11) includes an upper cap (111) and a lower cap (112), able to hermetically close the tank (11), opening and closing the lower cap (112) being controlled by rotating the aerosolising device (15), opening and closing the upper cap (111) being controlled by rotating the lower cap (112).

6. The device according to claim 5, **characterised in that** the lower cap (112) includes:
- a lower surface including a notch (117) designed to receive one end of the aerosolising device (158),
- an upper surface including a step (116) designed to control the upper cap (111), and
- a side wall including at least one locking lug (118).

7. The device according to any of claims 1 to 6, **characterised in that** the casing (13) includes a slide (132) to guide insertion and/or removal of the reservoir (11) onto or from said casing.

8. The device according to any of claims 1 to 7, **characterised in that** it comprises a label reader (25), connected to the control unit (20), for reading an identification label (115) affixed to the tank.

9. The device according to claim 8, **characterised in that** the identification label (115) includes a QR code and the label reader (25) is a QR code reader.

10. The device according to any of claims 1 to 9, **characterised in that** it comprises a device for detecting level or amount of the drug in the tank, connected to the control unit.

11. The device according to any of claims 1 to 10, **characterised in that** it comprises a rechargeable electric battery (14) housed in the casing (13) and supplying at least the control unit with electric energy.

12. The device according to any of claims 1 to 11, **characterised in that** it includes a display screen (22) for data relating to the administration of the drug and/or parametering data, connected to the control unit (20).
